(19) Europäisches Patentamt
European Patent Office
Office européen des brevets

(11) Veröffentlichungsnummer: **0 286 964 A2**

# (12) EUROPÄISCHE PATENTANMELDUNG

(21) Anmeldenummer: **88105494.4**

(51) Int. Cl.[4]: **C07C 147/02 , A01N 47/24**

(22) Anmeldetag: **07.04.88**

(30) Priorität: **14.04.87 DE 3712632**

(43) Veröffentlichungstag der Anmeldung: **19.10.88 Patentblatt 88/42**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal 20**
**D-5600 Wuppertal 1(DE)**
Erfinder: **Hänssler, Gerd, Dr.**
**Am Arenzberg 58a**
**D-5090 Leverkusen 3(DE)**
Erfinder: **Brandes, Wilhelm, Dr.**
**Eichendorffstrasse 3**
**D-5653 Leichlingen(DE)**

## (54) Alpha-Methylsulfonyl-benzaldoximcarbamate.

(57) α-Methylsulfonyl-benzaldoxim-carbamate der allgemeinen Formel (I)

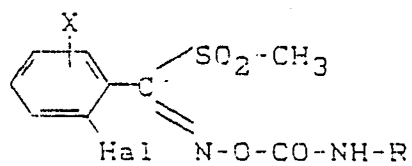

(I)

in welcher
R, X und Hal die in der Beschreibung gegebenen Bedeutungen haben und ihre Verwendung zur Bekämpfung von Schädlingen, vor allem von Pilzen.

Die neuen α-Methylsulfonyl-benzaldoxim-carbamate der allgemeinen Formel (I) können nach bekannten Verfahren aus geeigneten α-Methylsulfonyl-benzaldoximen, die ebenfalls noch neu und erfindungsgemäß sind, mit geeigneten Isocyanaten hergestellt werden. Die ebenfalls neuen α-Methylsulfonyl-benzaldoxime können aus geeigneten α-Halogenbenzaldoximen mit Methansulfinsäuren oder deren Alkalisalzen hergestellt werden.

EP 0 286 964 A2

## α-Methylsulfonyl-benzaldoxim-carbamate

Die vorliegende Erfindung betrifft neue α-Methylsulfonyl-benzaldoxim-carbamate, ein Verfahren zu ihrer Herstellung und ihre Verwendung zur Bekämpfung von Schädlingen.

Es sind bereits eine Reihe von Benzaldoximen, wie z.B das α-Phenylsulfonyl-2,6-dichlor-benzaldoxim, bekannt, ebenso deren Verwendung als Pflanzenschutzmittel; vor allem ist ihre Verwendung in Mitteln zur Bekämpfung von Weizensteinbrand (vgl. Schweizer Patent Nr. 423,350) bekannt.

Weiterhin sind α-Phenylsulfonyl-benzaldoxim-carbamate und ihre Verwendung im Pflanzenschutz bekannt (vgl. DE-OS 3,520,943).

Es wurden neue α-Methylsulfonyl-benzaldoxim-carbamate der allgemeinen Formel (I)

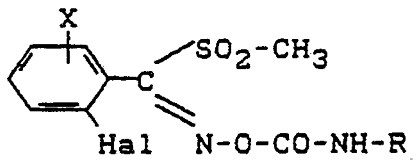

(I)

gefunden, in welcher

R für Alkyl mit 1 bis 6 Kohlenstoffatomen, für Halogenalkyl mit 1 bis 8 Kohlenstoffatomen und 1 bis 5 Halogenatomen, für Cyanalkyl mit 1 bis 6 Kohlenstoffatomen im Alkylteil, für Tosyl, für Benzyl, für gegebenenfalls ein-bis fünffach durch Alkyl mit 1 bis 4 Kohlenstoffatomen substituiertes Cycloalkyl mit 5 bis 7 Kohlenstoffatomen oder für gegebenenfalls ein-bis fünffach, gleich oder verschieden durch Alkyl mit 1 bis 6 Kohlenstoffatomen, Alkoxy mit 1 bis 4 Kohlenstoffatomen, Halogenalkyl oder Halogenalkoxy mit jeweils 1 bis 4 Kohlenstoffatomen und 1 bis 5 Halogenatomen und Halogen substituiertes Phenyl steht,

Hal für Halogen, wie Fluor, Chlor, Brom und Jod, steht und

X für Wasserstoff oder Halogen, wie Fluor, Chlor, Brom und Jod steht.

Weiterhin wurde gefunden, daß man die α-Methylsulfonyl-benzaldoxim-carbamate der allgemeinen Formel (I),

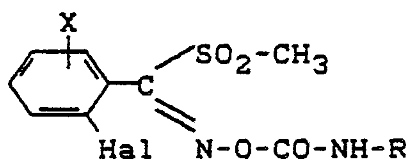

(I)

in welcher

R für Alkyl mit 1 bis 6 Kohlenstoffatomen, für Halogenalkyl mit 1 bis 8 Kohlenstoffatomen und 1 bis 5 Halogenatomen, für Cyanalkyl mit 1 bis 6 Kohlenstoffatomen im Alkylteil, für Tosyl, für Benzyl, für gegebenenfalls ein-bis fünffach durch Alkyl mit 1 bis 4 Kohlenstoffatomen substituiertes Cycloalkyl mit 5 bis 7 Kohlenstoffatomen oder für gegebenenfalls ein-bis fünffach, gleich oder verschieden durch Alkyl mit 1 bis 6 Kohlenstoffatomen, Alkoxy mit 1 bis 4 Kohlenstoffatomen, Halogenalkyl oder Halogenalkoxy mit jeweils 1 bis 4 Kohlenstoffatomen und 1 bis 5 Halogenatomen und Halogen substituiertes Phenyl steht,

Hal für Halogen steht und

X für Wasserstoff oder Halogen steht, erhält, wenn man α-Methylsulfonyl-benzaldoxime der allgemeinen Formel (II)

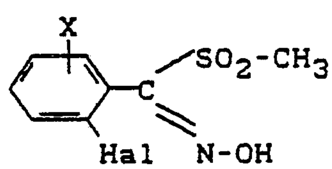

(II)

mit Isocyanaten der Formel (III)

R-NCO (III)

in welcher
R, Hal und X die oben angegebenen Bedeutungen haben,
gegebenenfalls in Gegenwart von Lösungs-oder Verdünnungsmittels bei Temperaturen von 0°C bis 100°C umsetzt.

Die erfindungsgemäßen $\alpha$-Methylsulfonylbenzaldoxim-carbamate der allgemeinen Formel (I) weisen starke biologische, vor allem fungizide Eigenschaften auf.

Überraschenderweise zeigen dabei die erfindungsgemäßen Verbindungen eine erheblich höhere, vor allem fungizide Wirksamkeit als die aus dem Stand der Technik bekannten Verbindungen, die strukturell und wirkungsmäßig sehr naheliegende Verbindungen sind.

Die erfindungsgemäßen Verbindungen der Formel (I) können als syn-oder anti-Isomere oder als deren Gemische in unterschiedlicher Zusammensetzung anfallen. Die Erfindung bezieht sich sowohl auf die reinen Isomeren als auch auf die Isomerengemische.

Die erfindungsgemäßen $\alpha$-Methylsulfonyl-benzaldoxim-carbamate sind durch die Formel (I) allgemein definiert. Bevorzugt sind Verbindungen der Formel (I), in welcher
R für geradkettiges oder verzweigtes Alkyl mit 1 bis 4 Kohlenstoffatomen, wie Methyl, Ethyl, n-Propyl, iso-Propyl, n-Butyl, iso-Butyl, sek.-Butyl und tert.-Butyl, für Halogenalkyl mit 1 bis 6 Kohlenstoffatomen und 1 bis 3 gleichen oder verschiedenen Halogenatomen, wie Fluor, Chlor, Brom und Iod, z.B. 2-Chlorethyl, 3-Chlor-n-propyl, 4-Chlor-n-butyl, 4-Chlor-n-pentyl und 6-Chlor-n-hexyl, für Cyanalkyl mit 1 bis 5 Kohlenstoffatomen, z.B. 3-oder 5-Cyan-n-pentyl, für Tosyl, für Benzyl, für gegebenenfalls ein-bis dreifach, gleich oder verschieden durch Alkyl mit 1 bis 3 Kohlenstoffatomen, wie Methyl, Ethyl, n-Propyl und iso-Propyl, substituiertes Cycloalkyl mit 6 oder 7 Kohlenstoffatomen, für ge gebenenfalls ein-bis dreifach, gleich oder verschieden durch Alkyl mit 1 bis 4 Kohlenstoffatomen, wie z.B. Methyl, Ethyl, n-Propyl, iso-Propyl, n-Butyl, iso-Butyl, sek.-Butyl und tert.-Butyl, durch Alkoxy mit 1 bis 3 Kohlenstoffatomen, wie Methoxy, Ethoxy, n-Propoxy und iso-Propoxy, durch Halogen-alkyl bzw. Halogenalkoxy mit jeweils 1 bis 3 Kohlenstoffatomen und 1 bis 3 gleichen oder verschiedenen Halogenatomen, wie Fluor, Chlor, Brom und Iod, wie z.B. Trifluormethyl, Trichlormethyl, Dichlorfluormethyl, 2-Chlorethyl, 2-Fluorethyl, Trifluormethoxy, Dichlorfluormethoxy, 2-Chlorethoxy und 2-Fluorethoxy und durch Halogen, wie Fluor, Chlor, Brom und Iod, substituiertes Phenyl steht,
Hal für Fluor oder Chlor steht und
X für Wasserstoff, Fluor oder Chlor steht.

Besonders bevorzugt sind Verbindungen der Formel (I), bei welchen
R für Methyl, Ethyl, n-Propyl, iso-Propyl, n-Butyl, iso-Butyl, tert.-Butyl, 6-Chlor-n-hexyl, 5-Cyano-n-pentyl, Tosyl, Benzyl, für ein-bis dreifach durch Methyl oder Ethyl substituiertes Cyclohexyl, für ein-bis dreifach, gleich oder verschieden durch Methyl, Ethyl, n-Propyl, iso-Propyl, Methoxy, Ethoxy, Trifluormethoxy, Chlor und Fluor substituiertes Phenyl steht,
Hal für Fluor oder Chlor steht und
X für Wasserstoff, Fluor oder Chlor steht.

Insbesondere seien Verbindungen der Formel (I) genannt, bei welchen
R für Methyl, Ethyl, n-Propyl, iso-Propyl, n-Butyl, iso-Butyl, tert.-Butyl, 6-Chlor-n-hexyl, 5-Cyano-n-pentyl, Benzyl, Tosyl, 3,5,5-Trimethyl-cyclohexyl, Cyclohexyl, 4-Methyl-cyclohexyl, 4-Trifluormethoxy-phenyl, 2-, 3-oder 4-Methyl-phenyl, 3-Chlor-4-methyl-phenyl, 3-Methyl-4-chlorphenyl, 3-oder 4-Chlorphenyl, 3,4-oder 3,5-Dichlor-phenyl, 3-Trifluormethyl-phenyl, 4-Ethoxy-phenyl, 3-Chlor-4-trifluormethylphenyl, 2,6-Dimethylphenyl oder 3,6-Di-isopropyl-phenyl steht,
Hal für Fluor oder Chlor steht und
X für Fluor oder Chlor steht.

Ganz besonders bevorzugt sind die Verbindungen der Formel (I), in der
R für Methyl, Ethyl, Tosyl, 4-Trifluormethoxy-phenyl oder 3,5,5-Trimethyl-cyclohexyl steht und
Hal und X die oben angegebenen Bedeutungen haben.

Alkyl, Halogenalkyl, Alkoxy, Halogenalkoxy und Cyanalkyl können jeweils geradkettig oder verzweigt sein im Alkylteil, wo sie auch genannt sind.

Verwendet man $\alpha$-Methylsulfonyl-2,6-dichlorbenzaldoxim und Methylisocyanat als Ausgangsstoffe, so läßt sich der Reaktionsablauf des erfindungsgemäßen Verfahrens durch das folgende Formelschema darstellen:

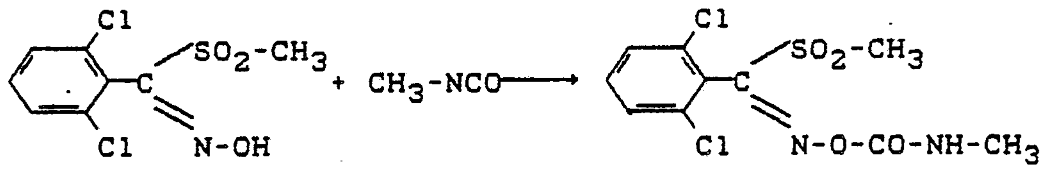

Die zur Durchführung des erfindungsgemäßen Verfahrens als Ausgangsstoffe benötigen α-Methylsulfonylbenzaldoxime sind durch die Formel (II) definiert. Diese Verbindungen sind neu und Teil dieser Erfindung. Sie können nach prinzipiell bekannten Verfahren hergestellt werden (vgl. z.B. CH 423 350 oder Tetrahedron 1975, 31 (6), 597-600), indem man α-Halogen-benzaldoxime der allgemeinen Formel (IV)

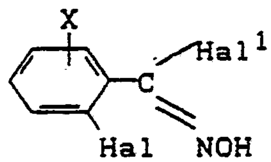

(IV)

in welcher
Hal' für Halogen, vorzugsweise für Chlor steht,
X und Hal die angegebenen Bedeutungen haben,
mit Methansulfinsäuren der allgemeinen Formel (V)

$CH_3$-$SO_2$M (V)

in welcher
M für Wasserstoff oder ein Alkalimetalläquivalent steht, gegebenenfalls in Gegenwart eines Lösungsmittels und gegebenenfalls in Gegenwart eines Säurebindemittels bei Temperaturen zwischen 0°C und 60°C umsetzt.

Auch die Verbindungen der Formel (II) sind nicht nur interessante Verbindungen für hoch wirksame Endprodukte, sondern zeigen auch selbst eine gute biologische Wirkung.

Das Verfahren zur Herstellung der Ausgangsverbindungen der Formel (II) kann gegebenenfalls in Gegenwart eines Lösungs-oder Verdünnungsmittels durchgeführt werden. Als solche kommen bevorzugt Alkohole, wie Methanol infrage.

Die Reaktionstemperaturen können in einem größeren Bereich variieren. Im allgemeinen arbeitet man zwischen 0 und 60°C, vorzugsweise zwischen 15 und 30°C.

Die α-Halogenbenzaldoxime der allgemeinen Formel (IV) können in bekannter Weise durch Halogenierung der entsprechenden Benzaldoxime gewonnen werden (vgl. z.B. CH 423,350 Beispiel I).

Die Alkansulfinsäuren der Formel (V) sind seit langem bekannte Grundchemikalien.

Die weiterhin als Ausgangsstoffe benötigten Isocyanate sind durch die Formel (III) allgemein definiert. Es handelt sich dabei um bekannte Verbindungen der organischen Chemie.

Das erfindungsgemäße Verfahren zur Herstellung der Verbindungen der Formel (I) kann gegebenenfalls in Gegenwart eines Lösungs-oder Verdünnungsmittels durchgeführt werden. Als solche kommen prinzipiell alle inerten organischen Lösungsmittel in Frage. Bevorzugt verwendet man Kohlenwasserstoffe, gegebenenfalls chlorierte, wie z.B. Benzol, Toluol, Xylol, Methylenchlorid, Chloroform, Tetrachlorkohlenstoff, Chlorbenzol und Dichlorbenzol.

Die Reaktionstemperaturen können in einem größeren Bereich variiert werden. Im allgemeinen arbeitet man zwischen 0 und 100°C, vorzugsweise zwischen 15 und 50°C.

Bei der Durchführung des erfindungsgemäßen Verfahrens läßt man die Reaktionspartner in etwa äquimolaren Verhältnissen miteinander reagieren. Da die Reaktion gelegentlich exotherm ist, empfiehlt es sich zu kühlen. Die Aufarbeitung des Reaktionsgemisches erfolgt in üblicher Weise, vornehmlich durch Absaugen, Waschen und Trocknen des ausgefallenen Reaktionsproduktes.

Zur Vervollständigung der Reaktion können gegebenenfalls einige Tropfen einer Base, wie z.B. Triethylamin, zugegeben werden.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch als Schädlingsbekämpfungsmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;

Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die erfindungsgemäßen Wirkstoffe lassen sich mit besonders gutem Erfolg zur Bekämpfung von Obstkrankheiten, verursacht z.B. von Venturia-Arten, oder von Reiskrankheiten, verursacht z.B. durch Pyricularia-Arten, verwenden. Erwähnenswert sind auch die gute Oomycetenwirkung und die bakterizide in-vitro-Wirkung.

Die Wirkstoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier-und/oder -schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90°.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-% vorzugsweise von 0,0001 bis 0,02 %, am Wirkungsort erforderlich.

Die erfindungsgemäßen Verbindungen der Formel (I) zeigen eine besonders gute Wirkung gegen Erreger von Obstkrankheiten, verursacht z.B. durch Venturia-Arten und Reiskrankheiten, verursacht durch Pyricularin-Arten. Ferner sei die gute Oomyceten-Wirkung und die in vitro-Wirkung gegen Bakterien erwähnt.

In entsprechenden Konzentrationen zeigen die erfindungsgemäßen Stoffe auch herbizide Wirkungen.

## Anwendungsbeispiele

In den nachfolgenden Anwendungsbeispielen werden die nachstehend aufgeführten Verbindungen als Vergleichssubstanzen verwendet:

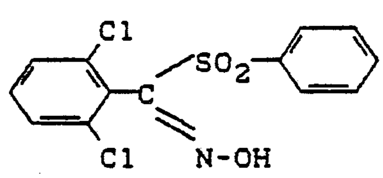

(A)

α-Phenylsulfonyl-2,6-dichlorbenzaldoxim (bekannt aus CH 423,350).

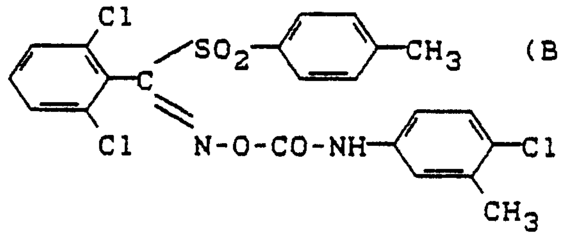

(B)

α-(4-Methylphenylsulfonyl)-2,6-dichlorbenzaldoxim-(4-chlor-3-methylphenyl)-carbamat

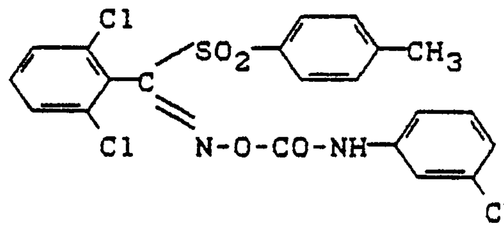

(C)

α-(4-Methylphenylsulfonyl)-2,6-dichlorbenzaldoxim-(3-methylphenyl)-carbamat

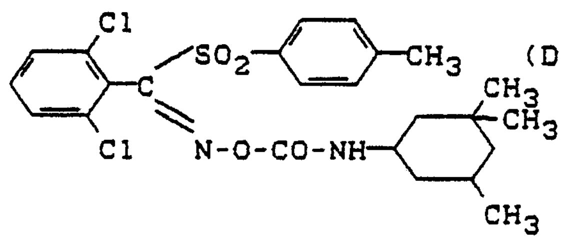

(D)

α-(4-Methylsulfonyl)-2,6-dichlorbenzaldoxim-(3,3,5-trimethylcyclohexyl)-carbamat
und

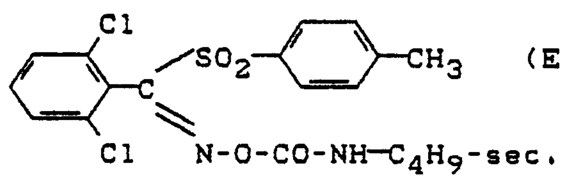

(E)

α-(4-Methylphenylsulfonyl)-2,6-dichlorbenzaldoxim-sec.-butyl-carbamat
(B-E bekannt aus DE-OS 3 520 943).

Beispiel A

Pyricularia-Test (Reis) /protektiv
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung

bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 6, 3, 1, 1A, 2A und 3A.

Beispiel B

Venturia-Test (Apfel) / protektiv
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1, 2, 6, 1A und 3A.

Herstellungsbeispiele

Beispiel 1:

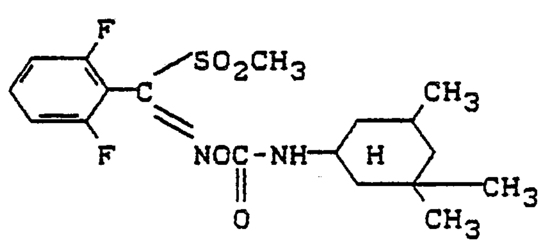

10 g (0,043 m) α-Methylsulfonyl-2,6-difluorbenzaldoxim werden in 150 ml Methylenchlorid suspendiert und mit 7,2 g (0,043 mol) 3,3,5-Trimethylcyclohexylisocyanat versetzt, wobei einige Tropfen Triethylamin zugesetzt werden. Man hält das Reaktionsgemisch über Nacht bei Raumtemperatur, es entsteht eine klare Lösung. Diese Lösung wird von einigen Verunreinigungen abfiltriert, anschließend im Vakuum eingeengt. Der amorphe Rückstand wird aus Isopropanol umkristallisiert. Man erhält 9,1 g (55% der Theorie) α-Methylsulfonyl-2,6-difluorbenzaldoxim-3,3,5-trimethylcyclohexylcarbamat vom Schmelzpunkt 152°C.

Analog Beispiel 1 und/oder den allgemeinen Angaben werden die Verbindungen der Formel (I)

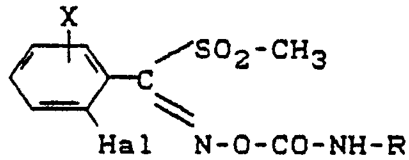

(I)

hergestellt.

| Beispiel Nr. | X 2-Stellg. | Hal | R | Schmelzpunkt [°C] |
|---|---|---|---|---|
| 2 | Cl | Cl | $C_2H_5$ | 184 |
| 3 | F | F | $C_2H_5$ | 116 |
| 4 | Cl | Cl | 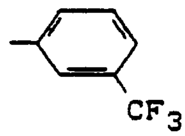 | 173 |
| 5 | Cl | Cl | 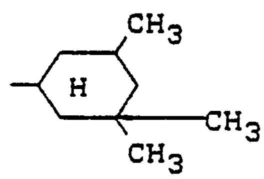 | 168 |
| 6 | Cl | Cl | $-(CH_2)_6-Cl$ | 90 |
| 7 | Cl | Cl | 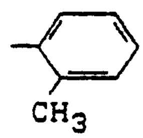 | |

| Beispiel Nr. | X 2-Stellg. | Hal | R | Schmelzpunkt [° C] |
|---|---|---|---|---|
| 8 | Cl | Cl | $CH_3$ | 158 |
| 9 | Cl | Cl | $C_3H_7$-n | 168 (Zers.) |
| 10 | Cl | Cl | $C_3H_7$-i | 150 (Zers.) |
| 11 | Cl | Cl | $C_4H_9$-n | 96 |
| 12 | Cl | Cl | $C_4H_9$-i | 123 |
| 13 | Cl | Cl | $C_4H_9$-sec. | 122 |
| 14 | Cl | Cl | 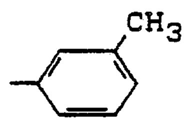 | 118 |
| 15 | Cl | Cl | 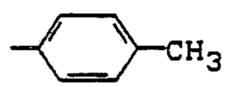 | 162 (Zers.) |
| 16 | Cl | Cl | 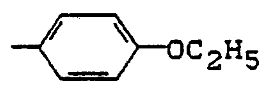 | 160 (Zers.) |
| 17 | Cl | Cl | 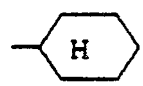 | 167 |
| 18 | Cl | Cl | 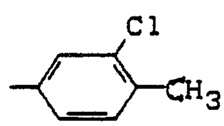 | 152 (Zers.) |

Herstellung der Ausgangsverbindungen

Beispiel 1 A:

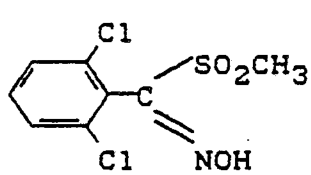

45 g (0,2 m) α-Chlor-2,6-Dichlorbenzaldoxim werden in 200 ml Methanol gelöst und mit 22,5 g (0,2 m) Methansulfinsäuren-Natriumsalz versetzt. Die Reaktion verläuft exotherm. Das Reaktionsgemisch wird über Nacht bei Raumtemperatur gerührt. Anschließend wird es auf ca. 1 l Eiswasser gegossen, ausgerührt,

abgesaugt, gewaschen und getrocknet. Nach dem Umkristallisieren aus Isopropanol erhält man 35,5 g (66% der Theorie) α-Methylsulfonyl-2,6-dichlorbenzaldoxim von Schmelzpunkt 193°C.

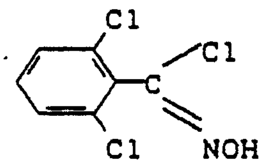

95 g (0,5 m) 2,6-Dichlórbenzaldoxim wurden in 1000 ccm Tetrachlorkohlenstoff suspendiert. In diese Suspension werden 36 g Chlor eingeleitet. Die Temperatur bleibt unter 15°C. Das Oxim muß dabei in Lösung gehen. Gleich anschließend wird die Lösung im Vakuum eingeengt. Der Rückstand kristallisiert größtenteils aus. Er wird aus heißem Cyclohexan umkristallisiert. Man erhält 50 g (45% der Theorie) α-Chlor-2,6-dichlorbenzaldoxim vom Fp. 114°C.

Analog dem Beispiel 1A oder den allgemeinen Angaben werden die Verbindungen der Formel (II)

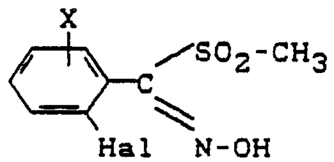

(II)

hergestellt:

| Beispiel Nr. | X 2-Stellg. | Hal | Schmelzpunkt [°C] |
|---|---|---|---|
| 2A | H | Cl | 161 |
| 3A | F | F | 153 |
| 4A | Cl | F | 168 |

## Ansprüche

1. α-Methylsulfonylbenzaldoxim-carbamate der allgemeinen Formel (I)

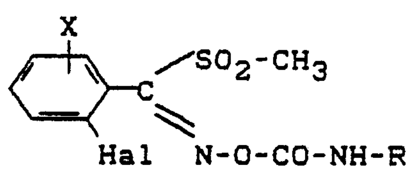

(I)

in welcher

R für Alkyl mit 1 bis 6 Kohlenstoffatomen, für Halogenalkyl mit 1 bis 8 Kohlenstoffatomen und 1 bis 5 Halogenatomen, für Cyanalkyl mit 1 bis 6 Kohlenstoffatomen im Alkylteil, für Tosyl, für Benzyl, für gegebenenfalls ein-bis fünffach durch Alkyl mit 1 bis 4 Kohlenstoffatomen substituiertes Cycloalkyl mit 5 bis 7 Kohlenstoffatomen oder für gegebenenfalls ein-bis fünffach, gleich oder verschieden durch Alkyl mit 1 bis 6 Kohlenstoffatomen, Alkoxy mit 1 bis 4 Kohlenstoffatomen, Halogenalkyl oder Halogenalkoxy mit jeweils 1 bis 4 Kohlenstoffatomen und 1 bis 5 Halogenatomen und Halogen substituiertes Phenyl steht,

Hal für Halogen steht, und

X für Wasserstoff oder Halogen, steht, deren Isomere und Isomerengemische.

2. α-Methylsulfonyl-benzaldoxim-carbamate gemäß Anspruch 1, worin in der Formel (I)
R für geradkettiges oder verzweigtes Alkyl mit 1 bis 4 Kohlenstoffatomen, für Halogenalkyl mit 1 bis 6 Kohlenstoffatomen und 1 bis 3 gleichen oder verschiedenen Halogenatomen, für Cyanalkyl mit 1 bis 5 Kohlenstoffatomen im Alkylteil, für Tosyl, für Benzyl, für gegebenenfalls ein-bis dreifach, gleich oder verschieden durch geradkettiges oder verzweigtes Alkyl mit 1 bis 3 Kohlenstoffatomen substituiertes Cycloalkyl mit 6 oder 7 Kohlenstoffatomen, für gegebenenfalls ein-bis dreifach, gleich oder verschieden durch geradkettiges oder verzweigtes Alkyl mit 1 bis 4 Kohlenstoffatomen, durch geradkettiges oder verzweigtes Alkoxy mit 1 bis 3 Kohlenstoffatomen, durch Halogenalkyl bzw. Halogenalkoxy mit jeweils 1 bis 3 Kohlenstoffatomen und 1 bis 3 Halogenatomen und durch Halogen substituiertes Phenyl steht,
Hal für Fluor oder Chlor steht und
X für Wasserstoff, Fluor oder Chlor steht, deren Isomere und Isomerengemische.

3. α-Methylsulfonyl-benzaldoxim-carbamate gemäß Anspruch 1, worin in der Formel (I)
R für Methyl, Ethyl, n-Propyl iso-Propyl, n-Butyl, iso-Butyl, tert.-Butyl, 6-Chlor-n-hexyl, 5-Cyano-n-pentyl, Tosyl, Benzyl, Cyclohexyl, für ein-bis dreifach durch Methyl oder Ethyl substituiertes Cyclohexyl, für ein-bis dreifach, gleich oder verschieden durch Methyl, Ethyl, n-Propyl, iso-Propyl, Methoxy, Ethoxy, Trifluormethoxy, Chlor und Fluor substituiertes Phenyl steht,
Hal für Fluor oder Chlor steht und
X für Wasserstoff, Fluor oder Chlor steht, deren Isomere und Isomerengemische.

4. α-Methylsulfonyl-benzaldoxim-carbamate gemäß Anspruch 1, worin in der Formel (I)
R für Methyl, Ethyl, n-Propyl, iso-Propyl, n-Butyl, iso-Butyl, tert.-Butyl, 6-Chlor-n-hexyl, 5-Cyano-n-pentyl, Benzyl, Tosyl, Cyclohexyl, 3,5,5-Trimethyl-cyclohexyl, 4-Methyl-cyclohexyl, 4-Trifluormethoxy-phenyl, 2-, 3- oder 4-Methyl-phenyl, 3-Chlor-4-methyl-phenyl, 3-Methyl-4-chlorphenyl, 3-oder 4-Chlorphenyl, 3,4-oder 3,5-Dichlor-phenyl, 3-Trifluormethyl-phenyl, 4-Ethoxy-phenyl, 3-Chlor-4-trifluormethylphenyl, 2,6-Dimethylphenyl oder 3,6-Di-isopropyl-phenyl steht,
Hal für Fluor oder Chlor steht und
X für Fluor oder Chlor steht, deren Isomere und Isomerengemische.

5. Verfahren zur Herstellung von α-Methylsulfonyl-benzaldoxim-carbamaten der Formel (I)

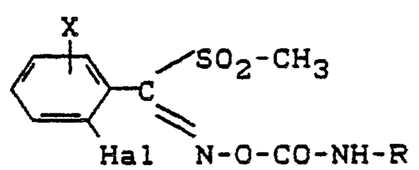

(I)

in welcher
R für Alkyl mit 1 bis 6 Kohlenstoffatomen, für Halogenalkyl mit 1 bis 8 Kohlenstoffatomen und 1 bis 5 Halogenatomen, für Cyanalkyl mit 1 bis 6 Kohlenstoffatomen im Alkylteil, für Tosyl, für Benzyl, für gegebenenfalls ein-bis fünffach durch Alkyl mit 1 bis 4 Kohlenstoffatomen substituiertes Cycloalkyl mit 5 bis 7 Kohlenstoffatomen oder für gegebenenfalls ein-bis fünffach, gleich oder verschieden durch Alkyl mit 1 bis 6 Kohlenstoffatomen, Alkoxy mit 1 bis 4 Kohlenstoffatomen, Halogenalkyl oder Halogenalkoxy mit jeweils 1 bis 4 Kohlenstoffatomen und 1 bis 5 Halogenatomen und Halogen substituiertes Phenyl steht,
Hal für Halogen steht und
X für Wasserstoff oder Halogen steht, deren Isomere und Isomerengemische, dadurch gekennzeichnet, daß man α-Methylsulfonyl-benzaldoxim der allgemeinen Formel (II)

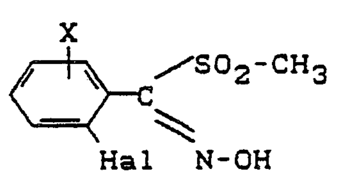

(II)

mit Isocyanaten der Formel (III)

R-NCO (III)

in welcher
R, Hal und X die oben angegebenen Bedeutungen haben,

gegebenenfalls in Gegenwart von Lösungs-oder Verdünnungsmitteln bei Temperaturen von 0°C bis 100°C umsetzt.

6. Schädlingsbekämpfungsmittel, gekennzeichnet durch einen Gehalt an mindestens einem α-Methylsulfonyl-benzaldoxim-carbamat der Formel (I) nach den Ansprüchen 1 und 5.

7. Verwendung von α-Methylsulfonyl-benzaldoxim-carbamaten der Formel (I) nach den Ansprüchen 1 und 5 zur Bekämpfung von Schädlingen.

8. Verfahren zur Bekämpfung von Schädlingen, dadurch gekennzeichnet, daß man α-Methylsulfonylbenzaldoxim-carbamate der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man α-Methylsulfonyl-benzaldoxim-carbamate der Formel (I) nach den Ansprüchen 1 und 5 mit Streckmitteln und/oder oberflächenaktiven Mitteln vermischt.

10. α-Methylsulfonyl-benzaldoxime der allgemeinen Formel (II)

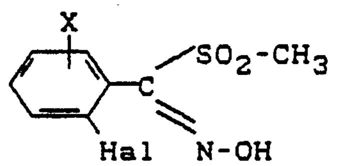

(II)

in welcher
Hal für Halogen und
X für Wasserstoff oder Halogen steht.

11. Verfahren zur Herstellung von α-Methylsulfonyl-benzaldoximen der Formel (II)

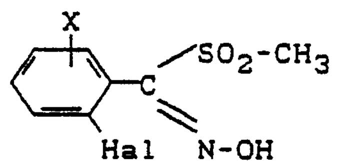

(II)

in welcher
Hal für Halogen und
X für Wasserstoff oder Halogen steht,
dadurch gekennzeichnet, daß man α-Halogen-benzaldoxime der allgemeinen Formel (IV)

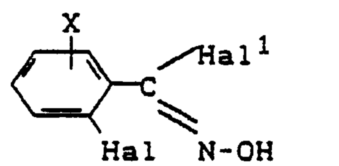

(IV)

in welcher
Hal¹ für Halogen, vorzugsweise für Chlor steht,
X und Hal die angegebenen Bedeutungen haben,
mit Methansulfinsäuren der allgemeinen Formel (V)

$CH_3\text{-}SO_2M$ (V)

in welcher
M für Wasserstoff oder ein Alkalimetalläquivalent steht, gegebenenfalls in Gegenwart eines Lösungsmittels und gegebenenfalls in Gegenwart eines Säurebindemittels bei Temperaturen zwischen 0°C und 60°C umsetzt.

12. Verwendung von α-Methylsulfonyl-benzaldoximen der Formel (II) nach den Ansprüchen 10 und 11 zur Bekämpfung von Schädlingen oder zur Synthese von Folgeprodukten zur Bekämpfung von Schädlingen.